(19) Europäisches Patentamt
European Patent Office
Office européen des brevets

(11) Veröffentlichungsnummer: **0 266 336 B1**

(12) **EUROPÄISCHE PATENTSCHRIFT**

(45) Veröffentlichungstag der Patentschrift: **15.05.91**

(51) Int. Cl.5: **C07D 333/32**, A61K 31/38, A61K 31/395

(21) Anmeldenummer: **87890040.6**

(22) Anmeldetag: **03.03.87**

Die Akte enthält technische Angaben, die nach dem Eingang der Anmeldung eingereicht wurden und die nicht in dieser Patentschrift enthalten sind.

(54) **Neue 1-/ 3-(2-Dialkylaminoäthoxy)-2-thienyl/-3-phenyl-1-propanone und ihre Säureadditionssalze und Verfahren zu deren Herstellung und sie enthaltende pharmazeutische Zusammensetzungen.**

(30) Priorität: **29.10.86 AT 2870/86**

(43) Veröffentlichungstag der Anmeldung:
**04.05.88 Patentblatt 88/18**

(45) Bekanntmachung des Hinweises auf die Patenterteilung:
**15.05.91 Patentblatt 91/20**

(84) Benannte Vertragsstaaten:
**AT BE CH DE ES FR GB IT LI LU NL SE**

(56) Entgegenhaltungen:
**EP-A- 0 053 603**
**DE-A- 3 316 155**

(73) Patentinhaber: **Binder, Dieter, Prof. Dr.**
**Sieveringerstrasse 207**
**A-1190 Wien(AT)**

(72) Erfinder: **Binder, Dieter, Prof. Dr.**
**Sieveringerstrasse 207**
**A-1190 Wien(AT)**

(74) Vertreter: **Pawloy, Heinrich, Dr. et al**
**Riemergasse 14**
**A-1010 Wien(AT)**

**EP 0 266 336 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue therapeutisch wertvolle 1-[3-(2-Dialkylaminoäthoxy)-2-thienyl]-3-phenyl-1-propanone der allgemeinen Formel

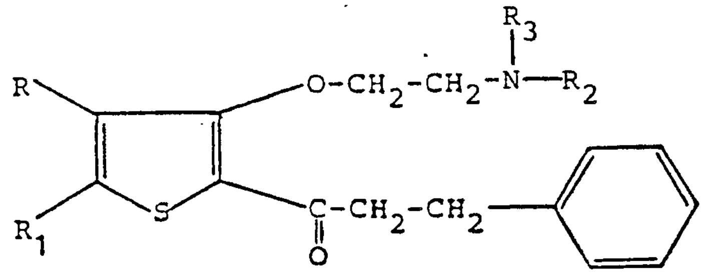

(I),

worin R und $R_1$ Wasserstoff oder Methyl sind und $R_2$ und $R_3$ gleich oder verschieden sind und jeweils Alkyl, Cycloalkyl, Alkenyl oder Alkinyl mit jeweils bis zu 8 C-Atomen bedeuten oder $R_2$ und $R_3$ zusammen mit dem sie verbindenden Stickstoffatom einen 5 bis 7-gliedrigen gesättigten heterocyclischen Ring bilden, der gegebenenfalls ein Sauerstoff- oder Stickstoffatom als weiteres Heteroatom im Ring enthalten kann, wobei ein zusätzliches Stickstoffatom durch einen Alkylrest mit 1 bis 3 C-Atomen substituiert sein kann, und ihre Säureadditionssalze, die antiarrhythmische Wirksamkeit aufweisen, ohne negativ inotrope Wirkung zu zeigen.

Die erfindungsgemäßen Verbindungen finden insbesondere Einsatz bei Erkrankungen des Herz-Kreislaufsystems. Aufgrund dieser pharmakologischen Eigenschaften können die neuen Verbindungen oder ihre Säureadditionssalze allein oder in Mischung mit anderen Wirksubstanzen in Form üblicher galenischer zubereitungen (auch Retardformen) bei Herzrhythumsstörungen als Medikament angewendet werden.

In der EP-A-0 053 603 sowie in DE-A-3 316 155 sind Verbindungen beschrieben, die sich von den erfindungsgemäßen Verbindungen dadurch unterscheiden, daß anstelle der 2-Dialkyl-amino-äthoxy-Seitenkette eine 2-Hydroxy-3-alkyl-aminopropoxy-Seitenkette vorhanden ist. Außerdem fehlt in den erfindungsgemäßen Verbindungen die ß-Blockergruppe, die in den Verbindungen der obgenannten Veröffentlichungen vorhanden ist.

Die Erfindung bezieht sich weiterhin auf ein Verfahren zur Herstellung von Verbindungen der Formel (I), welches dadurch gekennzeichnet ist, daß man eine Verbindung der allgemeinen Formel

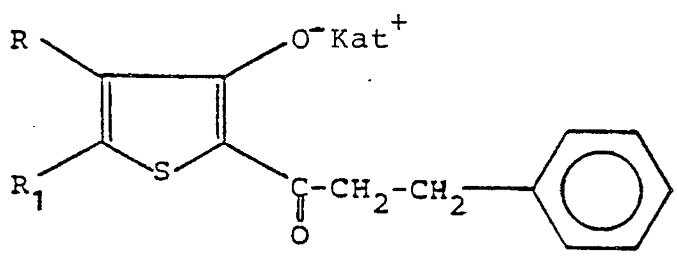

(II),

in der R und $R_1$ die oben genannte Bedeutung besitzen und $Kat^+$ ein Alkalimetall oder quartäres Ammoniumion ist, mit einer Verbindung der allgemeinen Formel

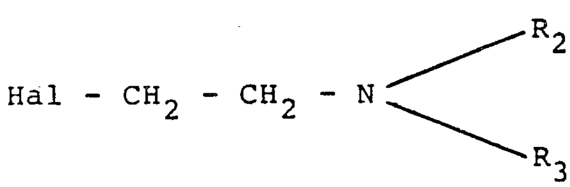

(III),

worin Hal Chlor, Jod oder Brom bedeutet und $R_2$ und $R_3$ die obige Bedeutung haben, in einem reaktionsinerten Lösungsmittel umsetzt und die erhaltenen Basen der Formel (I) gegebenenfalls in die Säureadditionssalze überführt.

Die erfindungsgemäße Umsetzung wird vorteilhafterweise so durchgeführt, daß man die korrespondierenden Säuren der Verbindungen der Formel (II) und die Verbindungen der Formel (III) in einem inerten organischen Lösungsmittel, vorzugsweise Toluol, Dimethylcarbonat, Diäthylcarbonat oder Ketonen, wie z.B. Methyläthylketon, in Gegenwart von überschüssigen, wasserfreien Alkalicarbonaten rückflußerhitzt. Bei höhersiedenden Lösungsmitteln soll aber die Temperatur von 130 °C nicht überschritten werden. Die Reaktionszeit hängt von der Reaktionstemperatur und Art des Lösungsmittels ab und kann zwischen 12 und 40 Stunden betragen.

Da die korrespondierenden Säuren der Verbindungen der Formel (II) relativ starke saure Eigenschaften besitzen, bilden sich unter den angegebenen Reaktionsbedingungen schon mit den Alkalicarbonaten die Alkalisalze dieser Verbindungen, die sich dann mit den Verbindungen der Formel (III) in einer Williams-Äther-Synthese umsetzen. Gemäß einer Verfahrensvariante ist es durchaus auch möglich, die Alkalisalze bzw. Ammoniumsalze mittels Alkalihydriden, Alkalihydroxiden, Alkalialkoholaten oder quartären Ammoniumhydroxiden separat herzustellen und diese dann in den angegebenen Lösungsmitteln mit Verbindungen der Formel (III) umzusetzen.

Da die freien Basen der Formel (I) meist nur schwer kristallisierende Öle sind, empfiehlt es sich, die Reinigung über gut kristallisierende Säureadditionsverbindungen, wie z.B. die Hydrochloride, vorzunehmen, die man gut umkristallisieren kann.

Diese Säureadditionssalze können dann in an sich bekannter Weise, z.B. mit Alkalien oder Ionenaustauschern in die freien Basen überführt werden, von denen sich durch Umsetzung mit anorganischen und organischen Säuren, insbesondere solchen, die zur Bildung von therapeutisch verwendbaren Salzen geeignet sind, weitere Salze gewinnen lassen.

Beispiele von Säuren, die mit den Verbindungen der Erfindung pharmazeutisch annehmbare Säureadditionssalze bilden, sind Salzsäure, Bromwasserstoffsäure, Phosphorsäure, Schwefelsäure, Citronensäure, Essigsäure, Bernsteinsäure, Maleinsäure, p-Toluolsulfonsäure und Methansulfonsäure. Es können jedoch auch andere Säuren verwendet werden.

Infolge der engen Beziehungen zwischen den neuen Verbindungen und deren Salzen sind im vorausgegangenen und nachfolgenden sinn- und zweckmäßig gegebenenfalls auch unter den freien Basen die entsprechenden Salze zu verstehen.

Die Ausgangsmaterialien der Formel (II) sind literaturbekannt (siehe z.B. AT-PS 369 739).

Verbindungen der Formel (III) sind, soweit sie nicht literaturbekannt sind, auf dem Fachmann bekannte Weise, ausgehend von den literaturbekannten Verbindungen (IV), herstellbar, z.B.

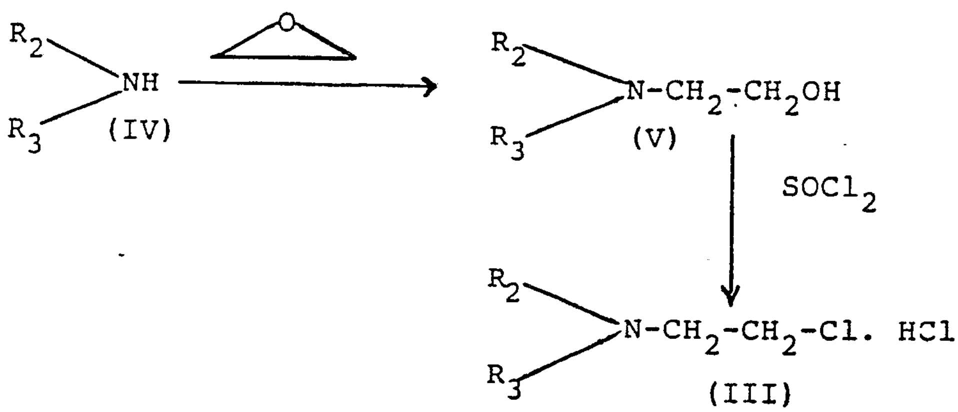


Unter den Arten von Tachykardien, die mit den erfindungsgemäßen Verbindungen behandelt werden können, seien supraventrikuläre Tachykardie, ventrikuläre Tachykardie, ventrikuläre Ektopie und "reentry" Tachykardie genannt.

Eine geeignete Dosis zur Verabreichung der neuen Verbindungen beträgt etwa 2 bis 10 mg/kg pro Tag, jedoch kommen je nach dem Zustand des zu behandelnden Patienten auch andere Dosen in Frage. Die neuen Verbindungen können in mehreren Dosen und auf oralem Weg verabreicht werden.

Die Erfindung bezieht sich schließlich auf pharmazeutische Zusammensetzungen, die eine wirksame Menge einer Verbindung der Formel (I) oder eines Säureadditionssalzes zusammen mit einem pharmazeutisch annehmbaren Träger aufweisen.

Die neuen Verbindungen können in den erfindungsgemäßen pharmazeutischen Zusammensetzungen in einem Anteil von etwa 100 bis 200 mg/Tablette vorhanden sein, wobei der Rest ein an sich bekannter pharmazeutisch annehmbarer Füllstoff ist.

Die pharmazeutischen Zusammensetzungen der Erfindung können außer in Form von Tabletten auch als Filmtabletten, Kapseln, Mikrokapseln, Zusammensetzungen mit verzögerter Freigabe des Wirkstoffes und anderen für orale Verabreichung geeigneten Formen vorliegen.

Die folgenden Beispiele sollen die vorliegende Erfindung näher erläutern, ohne daß diese hierauf beschränkt sein soll.

**Beispiel 1:**
1-[3-(2-Diäthylaminoäthoxy)-4-methyl-2-thienyl]-3-phenyl-1-propano -hydrochlorid (I) ($R_1$ = H, R = $CH_3$, $R_2$ und $R_3$ = Äthyl)

4,9 g 2-Diäthylaminoäthylchlorid-hydrochlorid werden zwischen 10 ml Toluol und 20 ml konz. Kaliumcarbonat-Lösung verteilt, die organische Phase abgetrennt, mit Natriumsulfat getrocknet und zu einer Lösung von 2 g (8,12 mMol) 1-(3-Hydroxy-4-methyl-2-thienyl)-1-propanon in 35 ml abs. Toluol zugegeben, diese Mischung mit 3,0 g wasserfreiem Kaliumcarbonat versetzt und unter Rühren 22 h rückflußerhitzt. Nach dem Abkühlen wird mit Wasser ausgeschüttelt, die organische Phase abgetrennt und mehrmals mit 2n HCl extrahiert. Die vereinigten salzsauren Phasen werden mit Natriumbicarbonat neutralisiert und mit Methylenchlorid mehrmals ausgeschüttelt. Die vereinigten Methylenchlorid-Phasen werden nun mit 2n HCl geschüttelt, die organische Phase abgetrennt, getrocknet und eingedampft. Das so verbleibende Hydrochlorid der Titelverbindung wird mit wenig Aceton zum Kristallisieren gebracht, auf -20 °C abgekühlt, abgesaugt und zweimal aus Aceton mit Aktivkohle umkristallisiert.

Ausbeute 1,63 g (52,6 %); Fp. 117 - 118 °C.

**Beispiel 2:**
1-[3-(2-Diäthylaminoäthoxy)-4-methyl-2-thienyl]-3-phenyl-1-propano -hydrochlorid

a) Freisetzen von 2-Diäthylaminoäthylchlorid
In einem 2 1 Erlenmeyerkolben werden 800 ml Diäthylcarbonat, 160,0 g (929,7 mMol) 2-Diäthylaminoäthylchlorid-hydrochlorid, 100 ml gesättigte $Na_2CO_3$-Lösung und 120 g $Na_2CO_3$ gerührt. Die Diäthylcarbonatphase wird in einen Scheidetrichter dekantiert, getrennt, mit $Na_2SO_4$ getrocknet und in einen Tropftrichter filtriert.

b) O-Alkylierung
28,5 g (115,7 mMol) 1-(3-Hydroxy-4-methyl-2-thienyl)-3-phenyl-1-propanon werden in 280 ml Diäthylcarbonat (DEC) gelöst, 120 ml (120 mMol) 1n NaOMe werden zugetropft und über eine kleine Brücke MeOH bis zu einer Sumpftemperatur von 90 °C abdestilliert. Man läßt das Reaktionsgemisch auf ca. 50 °C abkühlen und tropft nun die Lösung von 2-Diäthylaminoäthylchlorid in Diäthylcarbonat zu. Anschließend wird die Reaktionstemperatur 40 min bei 90 °C gehalten.

c) Aufarbeitung des freien Endproduktes
Das Reaktionsgemisch wird einrotiert und zwischen je 500 ml Äthylacetat und $NaHCO_3$-Lösung verteilt. Die wässerige Phase wird mit 2 x 200 ml Äthylacetat rückgeschüttelt.
Die organische Phase wird mit $Na_2SO_4$ getrocknet und mit einer Schaufel Aktivkohle gerührt und filtriert. Die Lösung wird einrotiert und abgesaugt, es verbleiben 37,5 g bernsteinfarbenes Öl (100 % der Theorie).

d) Umsetzung zum Hydrochlorid
Das Öl wird in je 200 ml $CHCl_3$ und Äther aufgenommen, nun wird trockene HCl durchgeleitet, bis die Lösung auf feuchtem pH-Papier sauer reagiert (25 min). Die Lösung wird einrotiert und abgesaugt, das entstehende Öl wird mit 50 ml Aceton auskristallisiert, 3 h im Tiefkühlschrank stehen gelassen, abgesaugt und mit 2 x 25 ml Aceton digeriert. Das Rohprodukt wird 2-mal aus Aceton (mit Aktivkohle) umkristallisiert; Ausbeute: 23,77 g (58,72 %).

Die erhaltene Verbindung entspricht der gemäß Beispiel 1 erhaltenen.

Nach dem obigen Verfahren können beispielsweise auch die in der folgenden Tabelle angegebenen Verbindungen der Formel (I) erhalten werden.

**TABELLE**

| R | $R_1$ | $R_2$ | $R_3$ |
|---|---|---|---|
| H, $CH_3$ | H, $CH_3$ | $CH_3$ | $CH_3$ |
| " | " | $CH_3$ | $CH_2-CH_3$ |
| " | " | $CH_3-CH_2$ | $CH_3-CH_2$ |
| " | " | $CH_3-CH_2$ | $CH_3-CH_2-CH_2-$ |
| " | " | $CH_3-CH_2-CH_2$ | $CH_3-CH_2-CH_2-$ |
| " | " | $-CH_2-CH=CH_2$ | $CH_3$ |
| " | " | $-CH_2-CH=CH_2$ | $CH_2-CH_3$ |
| " | " | $-CH_2-CH=CH_2$ | $-CH_2-CH=CH_2$ |
| " | " | $-(CH_2)_4-$ | |
| " | " | $-(CH_2)_5-$ | |
| " | " | $-(CH_2)_6-$ | |
| " | " | $-(CH_2)_2-O-(CH_2)_2-$ | |
| " | " | $-(CH_2)_2-N-(CH_2)_2-$<br>\|<br>$CH_3$ | |

**Beispiel 3:**

1-[3-(2-Diäthylaminoäthoxy)-4-methyl-2-thienyl]-3-phenyl-1-propano (I) ($R_1$ = H, R = $CH_3$, $R_2$ und $R_3$ = Äthyl)

2,0 g (5,24 mMol) 1-[3-(2-Diäthylaminoäthoxy)-4-methyl-2-thienyl]-3-phenyl-1-propanon-hyd ochlorid werden zwischen 20 ml gesättigter Natriumbicarbonatlösung und 20 ml Methylenchlorid verteilt, die organische Phase abgetrennt, getrocknet und eingedampft. Der ölige Rückstand wird bei 0,01 mbar und 185 °C (Luftbadtemperatur) kugelrohrdestilliert.
Ausbeute: 1,45 g (80 %) farbloses Öl.

**Beispiel 4 :**

1-[3-(2-Dimethylaminoäthoxy)-4-methyl-2-thienyl]-3-phenyl-1-propanon

34,5 g (0,24 Mol) 1-Chlor-2-dimethylaminothan-hydrochlorid werden mit einer gesättigten $Na_2CO_3$-Lösung on 108 g $Na_2CO_3$ (1 Mol) in 250 ml Wasser und 150 ml Diäthylcarbonat (DEC) 30 min gerührt. Nach Abdekantieren der Aminlösung wird nochmals mit 90 ml Diäthylcarbonat 10 min gerührt und die vereinten Aminlösungen werden über wasserfreiem Natriumsulfat und Natriumcarbonat getrocknet.

Die getrocknete Aminlösung wird zu 8,1 g (0,03 Mol) Natriumphenolat (hergestellt durch Auflösen von Phenol in der äquivalenten Menge methanolischer Natrium-Methylatlösung und nachfolgendes Eindampfen bis zur Gewichtskonstanz) zugesetzt und 45 min unter Rühren und Wasserausschluß (KOH Trockenrohr) auf 90 °C erhitzt. Nach Abkühlen der Lösung wird das Reaktionsgemisch zur Entfernung des ausgefallenen Salzes über eine Glasfritte abgesaugt und die Lösung im Vakuum eingedampft.

Der ölige Rückstand wird in 100 ml Methylenchlorid aufgenommen und 4-mal mit je 20 ml 0,5 n NaOH und 3-mal mit 10 ml Wasser ausgeschüttelt und 1-mal mit 10 ml Methylenchlorid rückgeschüttelt. Die vereinten $CH_2Cl$-Phasen werden intensiv mit 20 ml 4n HCl und 1-mal mit 5 ml Wasser geschüttelt, die wässerige Phase wird mit 5 ml Methylenchlorid rückgeschüttelt und die vereinten Methylenchloridphasen

werden über $Na_2SO_4$ wasserfrei getrocknet und eingedampft.

Das ölige Rohprodukt (13,2 g) wird in ca. 500 ml Aceton mit A ktivkohle behandelt. Man erhält 7,85 g (73,5 % bezogen auf Phenol) 1-[3-(2-Dimethylaminoäthoxy)-4-methyl-2-thienyl]-3-phenyl-1-propanonhydochlorid, Fp. 106 - 108° C (Aceton)

DC: Polygram SIL G/$UV_{254}$
Laufmittel: Toluol : Äthanol : konz. Ammoniak = 9 : 3 : 0,3

Endprodukt sichtbar: UV; Rf ca. 0,75
Amin sichtbar: $J_2$-Dämpfe
Phenol sichtbar: UV; 5 % $FeCl_3$ in 1n HCl

**Beispiel 5 :**

1-[3-(2-Piperidinyläthoxy)-4-methyl-2-thienyl]-3-phenyl-1-propanonydrochlorid

Es wird, wie in Beispiel 4 beschrieben, verfahren, wobei jedoch 15 g (0,082 Mol) 1-(2-Chloräthylpiperidin) (Hydrochlorid Fp. 230 - 233° C (Zers.) (sublim. 140 - 190° C); Lit. 230-233° C; F.H. Clarke, J.Org. Chem. 26, 1126-32 (1961)), zuerst 100 ml DEC und dann 30 ml DEC und 36 g (0,34 Mol) $Na_2CO_3$ in 100 ml Wasser verwendet werden.

Die getrocknete Aminlösung wird gemeinsam mit 2,7 g (0,010 Mol) Natriumphenolat 1 h unter Rühren und Feuchtigkeitsausschluß auf 90° C erhitzt.

Das Reaktionsgemisch wird, wie im Beispiel 4 beschrieben, aufgearbeitet und man erhält 3,25 g (82 %, bezogen auf Phenol) 1-[3-(2-Piperidinyläthoxy)-4-methyl-2-thienyl]-3-phenyl-1-propanon-hydrochlorid, Fp. 93 - 95° C (Aceton).

DC: Polygram SIL G/UV 0,67
Laufmittel: Toluol : Äthanol
: konz. Ammoniak = 9 : 3 : 0,3 Rf ca. 0,67

**Beispiel 6 :**

1-[3-(2-Pyrrolidyläthoxy)-4-methyl-2-thienyl]-3-phenyl-1-propanon-hydrochlorid

Es wird, wie in Beispiel 4 beschrieben, verfahren, wobei jedoch 13,6 g (0,08 Mol) 1-(2-Chloräthyl)-pyrrolidin-hydrochlorid (Fp. 173 - 174° C, ab 125° C zum Teil Sublimation und Umlagerung; Lit. 173,5 - 174° C; J. Am. Chem. Soc. 70 (1948), 3098-3100, J.B. Wright; Beilstein 20/IV/66), zuerst 100 ml DEC und dann 30 ml DEC und 37,5 g $Na_2CO_3$ in 100 ml Wasser verwendet werden.

Die getrocknete Aminlösung wird gemeinsam mit 2,7 g (0,010 Mol) Natriumphenolat 1 h unter Rühren und Feuchtigkeitsausschluß auf 90° C erhitzt.

Das Reaktionsgemisch wird, wie im Beispiel 4 beschrieben, aufgearbeitet und man erhält 2,1 g (55 %, bezogen auf Phenol) 1-[3-(2-Pyrrolidyläthoxy)-4-methyl-2-thienyl]-3-phenyl-1-propanon-hydrochlorid, Fp. 84 - 86 ° C (Aceton)

DC; Polygram SIL G/UV 254
Laufmittel: Toluol :
Äthanol : konz. Ammoniak = 9 : 3 : 0,3 Rf ca. 0,63

**Beispiel 7 :**

1-[3-(4-Morpholinyl)-äthoxy)-4-methyl-2-thienyl]-3-phenyl-1-propann-hydrochlorid

Es wird, wie in Beispiel 4 beschrieben, verfahren, wobei jedoch 15 g 4-(2-Chloräthyl)-morpholin-hydrochlorid (Fp. 184-185° C, Subl. ab 150° C, Lit. 178 - 180° C bzw. 182 - 182,5° C; F. Leonard, H. Horn; J. Am. Chem. Soc. 78, 1199-1201 (1956); J.P. Mason, H.W. Block; J. Am. Chem. Soc. 62, 1445 (1940)) in 100 ml Diäthylcarbonat und 35 g Natriumcarbonat in 250 ml Wasser verwendet werden.

Die getrocknete Aminlösung wird gemeinsam mit 2,7 g (0,01 Mol) Natriumphenolat 1 h unter Rühren und Feuchtigkeitsausschluß auf 90° C erhitzt. Die Lösung wird zur Entfernung des ausgefallenen Salzes über eine Glasfritte abgesaugt und die Lösung im Vakuum eingedampft. Der ölige Rückstand wird in 150

ml Methylenchlorid aufgenommen, 4-mal mit je 20 ml 0,5 n NaOH ausgeschüttelt sowie 3-mal mit 15 ml Wasser und 1-mal mit 10 ml Methylenchlorid rückgeschüttelt. Die über $Na_2SO_4$ getrocknete Methylenchloridphase ergibt 3,3 g öliges Rohprodukt. Die freie Base wird über eine mit Petroläther (40/60)-/Äther/Triäthylamin = 10/3/1 vorbehandelte Kieselgelsäule gereinigt.

Nach Elution mit Methylenchlorid, Einengen der Methylenchloridphase, Schütteln mit 4n HCl, Trocknen und Eindampfen erhält man 1,5 g 1-[3-(2-(4-Morpholinyl)-äthoxy)-4-methyl-2-thienyl]-3-phenyl-1-propanon-hydrochlorid (37,6 % der Theorie, bezogen auf Phenol), Fp. (Aceton -Diisopropyläther) 113 - 115° C DC/Rf = 0,65

Pharmakologische Eigenschaften der erfindungsgemäßen Verbindungen

Als repräsentative Verbindung wurde 1-[3-(2-Diäthylaminoäthoxy)-4-methyl-2-thienyl]-3-phenyl-1-propanon-hyd ochlorid hinsichtlich seiner antiarrhythmischen Wirksamkeit und der Wirkung auf den Kreislauf untersucht. Die Verlängerung der effektiven Refraktärperiode wurde als Kriterium zur Beurteilung der antiarrhythmischen Wirksamkeit herangezogen, die Wirkung auf die Kontraktionskraft war ein Parameter für die Kreislaufwirkung.

Als Vergleichssubstanzen dienten vor allem Chinidin und Lidocain als typische Antiarrhythmika mit Na-antagonistischer Wirkung.

Methode:

Die Untersuchungen wurden an isolierten linken Vorhöfen und Papillarmuskeln des rechten Ventrikels von Meerschweinchen mit einem Gewicht von ca. 250 - 500 g durchgeführt. Bei den Versuchen am Papillarmuskel wurde zur Organbadlösung eine Tyrode-Lösung mit hohem Kaliumgehalt gegeben. Die Messung der funktionellen Refraktärperiode (RP) wurde mittels der Doppelreizstimulierung (Govier 1975) durchgeführt. Dabei wurden Rechtecksimpulse mit einer Dauer von 3 ms und einer Basisfrequenz von 2 Hz angewandt. Zur Messung der effektiven RP wurden Doppelreize gesetzt, wobei auf den Grundstimulus ein identischer zweiter Reiz folgte, dessen zeitliche Verzögerung exakt variiert werden kann. Das Zeitintervall zwischen den beiden Impulsen wird beim Meßvorgang solange vergrößert, bis auch der zweite Stimulus mit einer Kontraktion der Vorhofmuskulatur beantwortet wird.

Der zeitliche Verlauf der Wirkung einzelner Dosen wurde verfolgt und für die Substanzen auch die Dosiswirkungskurve erstellt. Neben der Wirkung auf die effektive Refraktärperiode wurde gleichzeitig die Wirkung auf die Kontraktionskraft gemessen.

1-[3-(2-Diäthylaminoäthoxy)-4-methyl-2-thienyl]-3-phenyl-1-propano -hydrochlorid wurde in einer Konzentration von 10 µM, Chinidin und Lidocain in einer Konzentration von 100 µM dem Organbad zugesetzt. Zusätzlich wurde noch eine Kontrolle ohne Substanzzugabe mitlaufen lassen. Der Versuch am teilweise depolarisierten Papillarmuskel erfolgte in einem Organbad, das zusätzlich 22 mM Kalium enthielt, wodurch die schnellen Na-Kanälchen und nicht nur die langsamen Na-Ca-Kanälchen inaktiviert werden. Dieses Modell erscheint geeignet, die negativ inotrope Wirkung der am Vorhof getesteten Antiarrhythmika genauer zu untersuchen.

Ergebnis:

Chinidin (100 µM) bewirkte innerhalb von 30 min eine Verlängerung der RP um 161 %, Lidocain verlängerte in der gleichen Konzentration die RP lediglich um 63 %. Die Kontraktionskraft des Vorhofes wurde durch Chinidin innerhalb von 60 min auf 23 % gesenkt, während Lidocain eine Verminderung auf maximal 83 % verursachte.

1-[3-(2-Diäthylaminoäthoxy)-4-methyl-2-thienyl]-3-phenyl-1-propano -hydrochlorid (10 µM) verlängerte die RP des Vorhofes innerhalb von 60 min um 128 %, die Kontraktionskraft sank dabei nur geringfügig auf 89 % ab und entsprach damit der Kontraktionskraftsenkung, die auch unter Kontrollbedingungen, also ohne Substanzzugabe innerhalb von 60 min beobachtet wurde. Auffallend war, daß 1-[3-(2-Diäthylaminoäthoxy)-4-methyl-2-thienyl]-3-phenyl-1-propanon-hyd ochlorid bereits in einer Konzentration von 3 µM eine Verlängerung der RP um 73 % ohne gleichzeitige Senkung der Kontraktionskraft zeigte.

Am teilweise depolarisierten Papillarmuskel, bei dem die Wirkung auf eine Blockade von Na-Kanälchen verschiedener Substanzen untersucht werden kann, zeigte sich, daß sich in 1-[3-(2-Diäthylaminoäthoxy)-4-methyl-2-thienyl]-3-phenyl-1-propanonhydr chlorid eine vom Na-Antagonismus noch unabhängige Wirksamkeit entfaltet.

Zusammenfassung:

1-[3-(2-Diäthylaminoäthoxy)-4-methyl-2-thienyl]-3-phenyl-1-propano -hydrochlorid zeichnet sich durch ein besonders günstiges Verhältnis seine r antiarrhythmischen Wirkung zur negativ inotropen Wirkung aus. Es vermindert in einer Konzentration von 10 $\mu$M die Erregbarkeit und bewirkt eine Verlängerung der Refraktärperiode, wobei die Kontraktionskraft des Vorhofes wenig gehemmt wird. Am teilweise depolarisierten Papillarmuskel konnte überdies gezeigt werden, daß die Wirkung von 1-[3-(2-Diäthylaminoäthoxy)-4-methyl-2-thienyl]-3-phenyl-1-propanon-hyd ochlorid auch in einer vom Angriff am Na-Kanal unabhängigen Wirkung bestehen dürfte. Mit dieser Wirkungscharakteristik unterscheidet sich 1-[3-(2-Diäthylaminoäthoxy)-4-methyl-2-thienyl]-3-phenyl-1-propanon-hyd ochlorid grundlegend von den Vergleichssubstanzen.

**Ansprüche**

1. 1-[3-(2-Dialkylaminoäthoxy)-2-thienyl]-3-phenyl-1-propanone der allgemeinen Formel

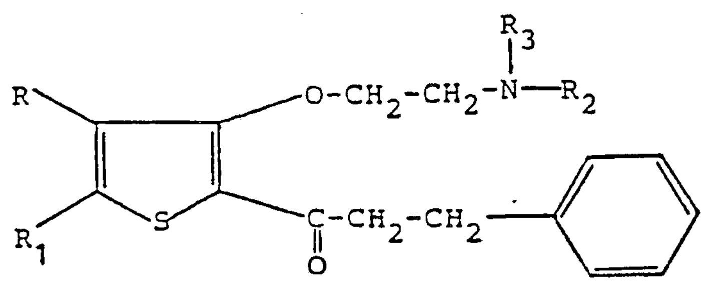

(I),

worin R und $R_1$ Wasserstoff oder Methyl sind und $R_2$ und $R_3$ gleich oder verschieden sind und jeweils Alkyl, Cycloalkyl, Alkenyl oder Alkinyl mit jeweils bis zu 8 C-Atomen bedeuten oder $R_2$ und $R_3$ zusammen mit dem sie verbindenden Stickstoffatom einen 5 bis 7-gliedrigen gesättigten heterocyclischen Ring bilden, der gegebenenfalls ein Sauerstoff- oder Stickstoffatom als weiteres Heteroatom im Ring enthalten kann, wobei ein zusätzliches Stickstoffatom durch einen Alkylrest mit 1 bis 3 C-Atomen substituiert sein kann, und ihre Säureadditionssalze.

2. 1-[3-(2-Diäthylaminoäthoxy)-4-methyl-2-thienyl]-3-phenyl-1-propanon-hydrochlorid.

3. 1-[3-(2-Dimethylaminoäthoxy)-4-methyl-2-thienyl]-3-phenyl-1-propanon.

4. 1-[3-(2-Piperidinyläthoxy)-4-methyl-2-thienyl]-3-phenyl-1-propanon-hydro chlorid.

5. 1-[3-(2-Pyrrolidyläthoxy)-4-methyl-2-thienyl]-3-phenyl-1-propanon-hydroch lorid.

6. 1-[3-(2-4-Morpholinyl)-äthoxy)-4-methyl-2-thienyl]-3-phenyl-1-propanon-hydrochlorid.

7. Verfahren zur Herstellung von 1-[3-(2-Dialkylaminoäthoxy)-2-thienyl]-3-phenyl-1-propanonen der Formel (I), wie in Anspruch 1 definiert, und deren Säureadditionssalze , dadurch gekennzeichnet, daß man eine Verbindung der allgemeinen Formel

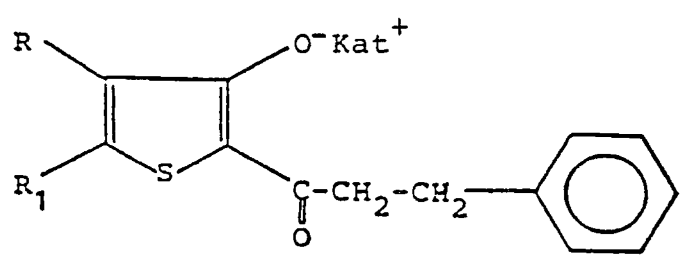

(II),

in der R und $R_1$ die oben genannte Bedeutung besitzen und $Kat^+$ ein Alkalimetall- oder quartäres

Ammoniumion ist, mit einer Verbindung der allgemeinen Formel

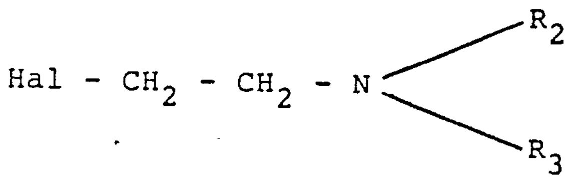

(III),

worin Hal Chlor, Jod oder Brom bedeutet und $R_2$ und $R_3$ die obige Bedeutung haben, in einem reaktionsinerten Lösungsmittel umsetzt und die erhaltenen Basen der Formel (I) gegebenenfalls in die Säureadditionssalze überführt.

**8.** Pharmazeutische Zusammensetzung, dadurch gekennzeichnet, daß sie eine Verbindung nach einem der Ansprüche 1 bis 6 oder ein pharmazeutisch annehmbares Säureadditionssalz hievon zusammen mit einem pharmazeutisch annehmbaren Träger, gegebenenfalls in Mischung mit anderen Wirksubstanzen, enthält.

**9.** Verwendung von Verbindungen der Formel (I) und deren Säureadditionssalzen für die Herstellung von Medikamenten zur Behandlung von Herzrhythmusstörungen.

Patentansprüche für folgenden Vertragstaat: ES

**1.** Verfahren zur Herstellung von 1-[3-(2-Dialkylaminoäthoxy)-2-thienyl]-3-phenyl-1-propanonen der allgemeinen Formel

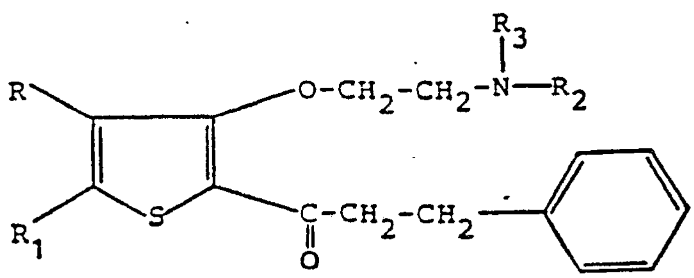

(I),

worin R und $R_1$ Wasserstoff oder Methyl sind und $R_2$ und $R_3$ gleich oder verschieden sind und jeweils Alkyl, Cycloalkyl, Alkenyl oder Alkinyl mit jeweils bis zu 8 C-Atomen bedeuten oder $R_2$ und $R_3$ zusammen mit dem sie verbindenden Stickstoffatom einen 5 bis 7-gliedrigen gesättigten heterocyclischen Ring bilden, der gegebenenfalls ein Sauerstoff- oder Stickstoffatom als weiteres Heteroatom im Ring enthalten kann, wobei ein zusätzliches Stickstoffatom durch einen Alkylrest mit 1 bis 3 C-Atomen substituiert sein kann, und ihrer Säureadditionssalze, dadurch gekennzeichnet, daß man eine Verbindung der allgemeinen Formel

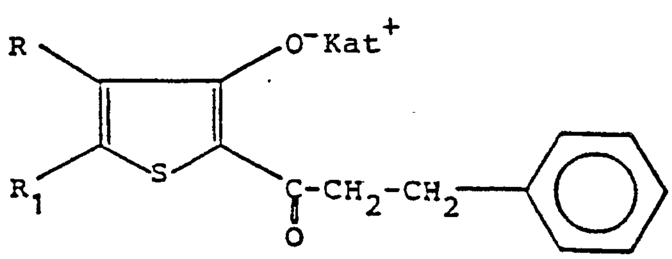

(II),

in der R un d $R_1$ die oben genannte Bedeutung besitzen und $Kat^+$ ein Alkalimetall oder quartäres Ammoniumion ist, mit einer Verbindung der allgemeinen Formel

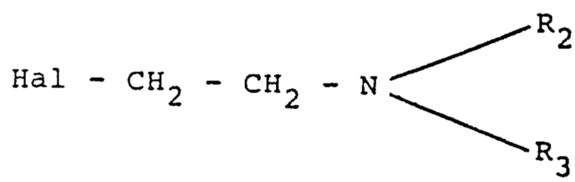

(III),

worin Hal Chlor, Jod oder Brom bedeutet und $R_2$ und $R_3$ die obige Bedeutung haben, in einem reaktionsinerten Lösungsmittel umsetzt und die erhaltenen Basen der Formel (I) gegebenenfalls in die Säureadditionssalze überführt.

2. Verwendung von Verbindungen der Formel (I) und deren Säureadditionssalzen für die Herstellung von Medikamenten zur Behandlung von Herzrhythmusstörungen.

## Claims

1. 1-[3-(2-Dialkylaminoethoxy)-2-thienyl]-3-phenyl-1-propanones of the general formula

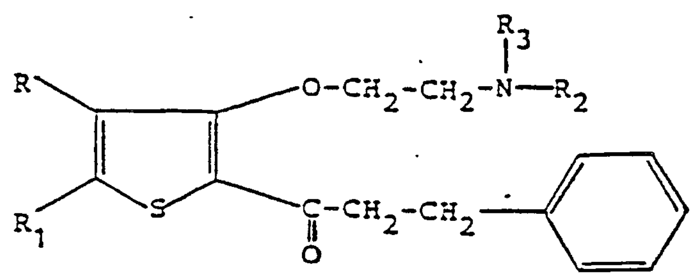

(I),

in which R and $R_1$ are hydrogen or methyl and $R_2$ and $R_3$ are identical or different and in each case denote alkyl, cycloalkyl, alkenyl or alkynyl in each case having up to 8 C atoms or $R_2$ and $R_3$, together with the nitrogen atom bonding them, form a 5 to 7-membered saturated heterocyclic ring which may optionally contain an oxygen or nitrogen atom as a further heteroatom in the ring, it being possible to substitute an additional nitrogen atom by an alkyl radical having 1 to 3 C atoms, and their acid addition salts.

2. 1-[3-[2-Diethylaminoethoxy)-4-methyl-2-thienyl]-3-phenyl-1-propanone hydrochloride.

3. 1-[3-(2-Dimethylaminoethoxy)-4-methyl-2-thienyl]-3-phenyl-1-propanone.

4. 1-[3-(2-Piperidinylethoxy)-4-methyl-2-thienyl]-3-phenyl-1-propanone hydrochloride.

5. 1-[3-(2-Pyrrolidylethoxy)-4-methyl-2-thienyl]-3-phenyl-1-propanone hydrochloride.

6. 1-[3-(2-(4-Morpholinyl)-ethoxy)-4-methyl-2-thienyl]-3-phenyl-1-propanone hydrochloride.

7. Process for the preparation of 1-[3-(2-dialkyl-aminoethoxy)-2-thienyl]-3-phenyl-1-propanones of the formula (I), as defined in Claim 1, and their acid addition salts, characterised in that a compound of the general formula

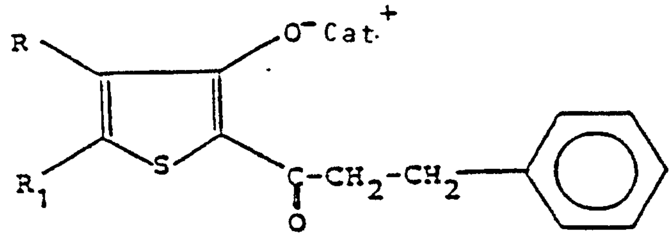

(II),

in which R and $R_1$ have the abovementioned meaning and $Cat^+$ is an alkali metal or quaternary ammonium ion, is reacted with a compound of the general formula

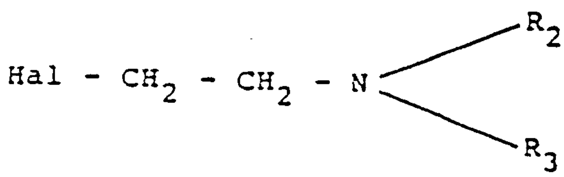

(III),

in which Hal is chlorine, iodine or bromine and $R_2$ and $R_3$ have the abovementioned meaning, in a solvent which is inert to the reaction and the bases of the formula (I) obtained are optionally converted into the acid addition salts.

**8.** Pharmaceutical composition, characterised in that it contains a compound according to one of Claims 1 to 6 or a Pharmaceutically acceptable acid addition salt thereof together with a pharmaceutically acceptable carrier, if desired mixed with other active substances.

**9.** Use of compounds of the formula (I) and their acid addition salts for the production of medicaments for the treatment of cardiac arrhythmias.

Claims for the following contracting state: ES

**1.** Process for the preparation of 1-[3-(2-dialkyl-aminoethoxy)-2-thienyl]-3-phenyl-1-propanones of the general formula

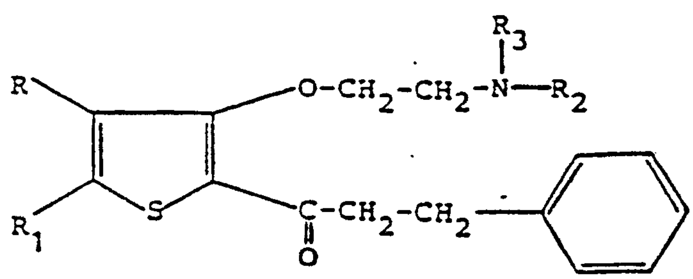

(I),

in which R and $R_1$ are hydrogen or methyl and $R_2$ and $R_3$ are identical or different and in each case denote alkyl, cycloalkyl, alkenyl or alkynyl in each case having up to 8 C atoms or $R_2$ and $R_3$, together with the nitrogen atom bonding them, form a 5 to 7-membered saturated heterocyclic ring which may optionally contain an oxygen or nitrogen atom as a further heteroatom in the ring, it being possible to substitute an additional nitrogen atom by an alkyl radical having 1 to 3 C atoms, and their acid addition salts, characterised in that a compound of the general formula

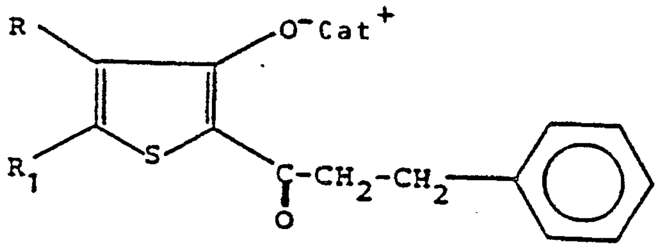

(II),

in which R and $R_1$ have the abovementioned meaning and $Cat^+$ is an alkali metal or quaternary ammonium ion, is reacted with a compound of the general formula

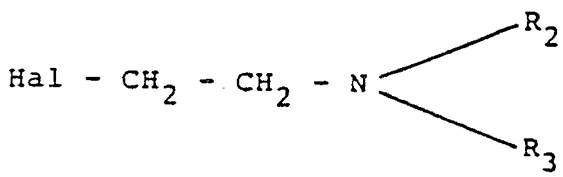

(III),

in which Hal is chlorine, iodine or bromine and $R_2$ and $R_3$ have the abovementioned meaning, in a solvent which is inert to the reaction and the bases of the formula (I) obtained are optionally converted into the acid addition salts.

**2.** Use of compounds of the formula (I) and their acid addition salts for the production of medicaments for the treatment of cardiac arrhythmias.

**Revendications**

**1.** 1-[3-(2-dialkylaminoéthoxy)-2-thiényl]-3-phényl-1-propanones de formule générale

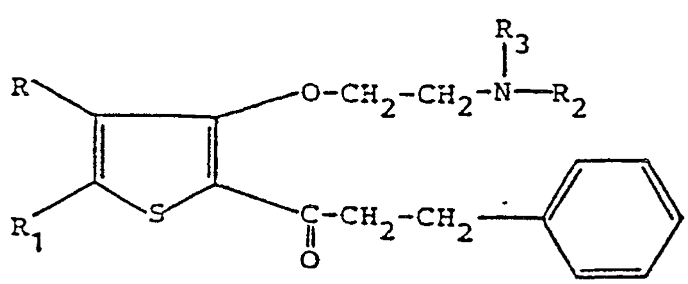

(I)

dans laquelle R et $R_1$ représentent l'hydrogène ou des groupes méthyle et $R_2$ et $R_3$ sont identiques ou différents et représentent chacun un groupe alkyle, cycloalkyle, alcényle ou alcynyle ayant chacun jusqu'à 8 atomes de carbone, ou bien $R_2$ et $R_3$ forment conjointement avec l'atome d'azote auquel ils sont liés un noyau hétérocyclique saturé pentagonal à heptagonal, qui peut contenir, le cas échéant, un atome d'oxygène ou d'azote comme hétéroatome supplémentaire dans le noyau, un atome d'azote supplémentaire pouvant être substitué par un reste alkyle de 1 à 3 atomes de carbone et leurs ses sels d'addition d'acides.

**2.** Chlorhydrate de 1-[3-(2-diéthylaminoéthoxy)-4-méthyl-2-thiényl]-3-phényl-1-propanone.

**3.** 1-[3-(2-diméthylaminoéthoxy)-4-méthyl-2-thiényl]-3-phényl-1-propanone.

**4.** Chlorhydrate de 1-[3-(2-pipéridinyléthoxy)-4-méthyl-2-thiényl]-3-phényl-1-propanone.

**5.** Chlorhydrate de 1-[3-(2-pyrrolidyléthoxy)-4-méthyl-2-thiényl]-3-phényl-1-propanone.

**6.** Chlorhydrate de 1-[3-(2-(4-morpholinyl)-éthoxy)-4-méthyl-2-thiényl]-3-phényl-1-propanone.

7. Procédé de production de 1-[3-(2-dialkyl-aminoéthoxy)-2-thiényl]-3-phényl-1-propanones de formule (I), telle que définie dans la revendication 1, et de leurs sels d'addition d'acides, caractérisé en ce qu'on fait réagir un composé de formule générale

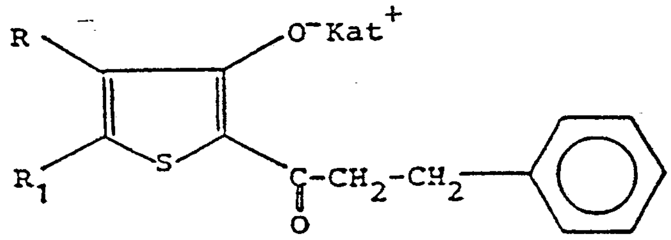

(II)

dans laquelle R et $R_1$ ont la définition indiquée ci-dessus et $Kat^+$ désigne un ion de métal alcalin ou un ion d'ammonium quaternaire, avec un composé de formule générale

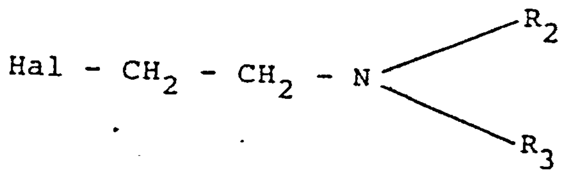

(III)

dans laquelle Hal désigne le chlore, l'iode ou le brome et $R_2$ et $R_3$ ont la définition indiquée ci-dessus, dans un solvant inerte vis-à-vis de la réaction, et on transforme éventuellement les bases obtenues de formule (I) en les sels d'addition d'acides.

8. Composition pharmaceutique, caractérisée en ce qu'elle contient un composé suivant l'une des revendications 1 à 6 ou un sel d'addition d'acide pharmaceutiquement acceptable de ce composé en association avec un support acceptable du point de vue pharmaceutique, éventuellement en mélange avec d'autres substances actives.

9. Utilisation de composés de formule (I) et de leurs sels d'addition d'acides pour la préparation de médicaments destinés au traitement de troubles du rythme cardiaque.

Revendications pour l'Etat contractant suivant: ES

1. Procédé de préparation de 1-[3-(2-dialkyl-aminoéthoxy)-2-thiényl]-3-phényl-1-propanones de formule générale

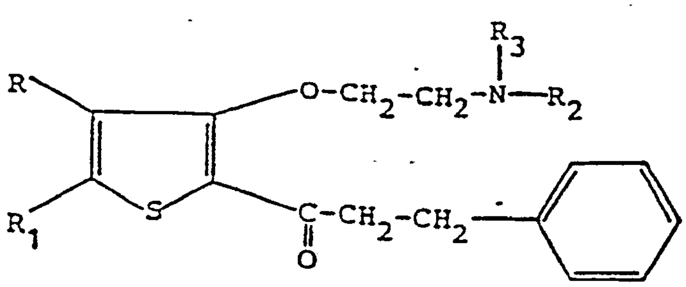

(I)

dans laquelle R et $R_1$ représentent l'hydrogène ou des groupes méthyle et $R_2$ et $R_3$ sont identiques ou différents et représentent chacun un groupe alkyle, cycloalkyle, alcényle ou alcynyle ayant chacun jusqu'à 8 atomes de carbone, ou bien $R_2$ et $R_3$ forment conjointement avec l'atome d'azote auquel ils sont liés un noyau hétérocyclique saturé pentagonal à heptagonal, qui peut contenir, le cas échéant, un atome d'oxygène ou d'azote comme hétéroatome supplémentaire dans le noyau, un atome d'azote supplémentaire pouvant être substitué par un reste alkyle de 1 à 3 atomes de carbone, et de leurs sels d'addition d'acides, caractérisé en ce qu'on fait réagir un composé de formule générale

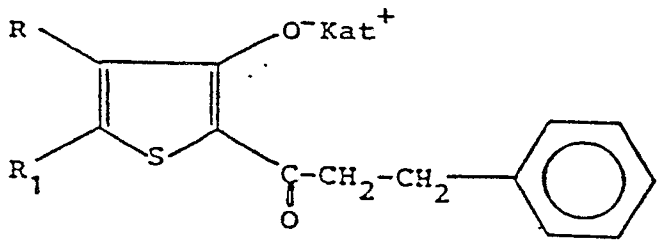

(II).

dans laquelle R et $R_1$ ont la définition indiquée ci-dessus et $Kat^+$ désigne un ion de métal alcalin ou un ion d'ammonium quaternaire, avec un composé de formule générale

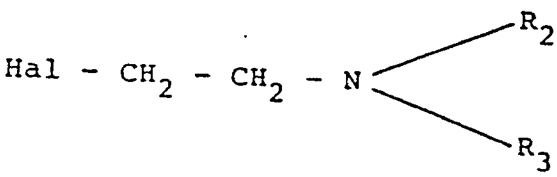

(III)

dans laquelle Hal désigne le chlore, l'iode ou le brome et $R_2$ et $R_3$ ont la définition indiquée ci-dessus, dans un solvant inerte vis-à-vis de la réaction, et on transforme éventuellement les bases obtenues de formule (I) en les sels d'addition d'acides.

2. Utilisation de composés de formule (I) et de leurs sels d'addition d'acides pour la préparation de médicaments destinés au traitement de troubles du rythme cardiaque.